# FASCICULE DE BREVET EUROPEEN

(11) **EP 3 996 676 B1**
(45) Date de publication et mention de la délivrance du brevet: **26.03.2025**
(21) Numéro de dépôt: 20751666.7
(22) Date de dépôt: 10.07.2020
(51) Int. Cl.: A61K 31/12, A61K 9/00, A61K 9/06, A61K 9/14, A61K 31/717, A61K 31/724, A61P 25/02, A61K 36/9066

(54) **POTENTIEL THÉRAPEUTIQUE DES NANOCRISTAUX DE CELLULOSE-CYCLODEXTRINE-CURCUMINE DANS LE TRAITEMENT DES NEUROPATHIES PÉRIPHÉRIQUES**
THERAPEUTISCHES POTENTIAL VON CURCUMIN/CYCLODEXTRIN/ZELLULOSENANOKRISTALLEN BEI DER BEHANDLUNG PERIPHERER NEUROPATHIEN
THERAPEUTIC POTENTIAL OF CURCUMIN/CYCLODEXTRIN/CELLULOSE NANOCRYSTALS IN THE TREATMENT OF PERIPHERAL NEUROPATHIES

(30) Priorité: 12.07.2019 FR 1907897
(43) Date de publication de la demande: 18.05.2022
(73) Titulaire: Université de Limoges, 87032 Limoges (FR)
(72) Inventeur: CAILLAUD, Martial, 87025 Limoges CEDEX (FR); DESMOULIÈRE, Alexis, 87025 Limoges CEDEX (FR); BILLET, Fabrice, 87025 Limoges CEDEX (FR); STURTZ, Franck, 87025 Limoges CEDEX (FR); NDONG-NTOUTOUME, Gautier Marck Arthur, 87060 Limoges CEDEX (FR); SOL, Vincent, 87060 Limoges CEDEX (FR); GRANET, Robert, 87060 Limoges CEDEX (FR)
(74) Mandataire: Plasseraud IP
(86) Numéro de dépôt international: PCT/IB2020/056505
(87) Numéro de publication internationale: WO 2021/009640

(56) Documents cités:
- HIROKI SASAKI ET AL: "Innovative Preparation of Curcumin for Improved Oral Bioavailability", BIOLOGICAL & PHARMACEUTICAL BULLETIN, vol. 34, no. 5, 1 January 2011 (2011-01-01), JP, pages 660 - 665, XP055444380, ISSN: 0918-6158, DOI: 10.1248/bpb.34.660
- YAVARPOUR-BALI HANIE ET AL: "Curcumin-loaded nanoparticles: a novel therapeutic strategy in treatment of central nervous system disorders", INTERNATIONAL JOURNAL OF NANOMEDICINE, vol. Volume 14, 17 June 2019 (2019-06-17), New Zealand, pages 4449 - 4460, XP093209052, ISSN: 1178-2013, Retrieved from the Internet <URL:https://www.dovepress.com/getfile.php?fileID=50542> DOI: 10.2147/IJN.S208332
- CAILLAUD MARTIAL ET AL: "Local low dose curcumin treatment improves functional recovery and remyelination in a rat model of sciatic nerve crush through inhibition of oxidative stress", NEUROPHARMACOLOGY, ELSEVIER, AMSTERDAM, NL, vol. 139, 3 July 2018 (2018-07-03), pages 98 - 116, XP085430148, ISSN: 0028-3908, DOI: 10.1016/J.NEUROPHARM.2018.07.001
- NDONG NTOUTOUME GAUTIER M A ET AL: "Development of curcumin-cyclodextrin/cellulose nanocrystals complexes: New anticancer drug delivery systems", BIOORGANIC & MEDICINAL CHEMISTRY LETTERS, PERGAMON, AMSTERDAM, NL, vol. 26, no. 3, 18 December 2015 (2015-12-18), pages 941 - 945, XP029391914, ISSN: 0960-894X, DOI: 10.1016/J.BMCL.2015.12.060
- SHERER: "2018 Peripheral Nerve Society Annual Meeting 21-25 July, 2018 Baltimore, Maryland", JOURNAL OF THE PERIPHERAL NERVOUS SYSTEM, vol. 23, no. 4, 1 December 2018 (2018-12-01), US, pages 249 - 405, XP055676584, ISSN: 1085-9489, DOI: 10.1111/jns.12290

## Description

### ARRIERE-PLAN DE L'INVENTION

La présente invention concerne le domaine du traitement de tout type de neuropathies périphériques, en particulier de la maladie de Charcot-Marie-Tooth 1A.

Les nerfs périphériques sont sujets à de nombreuses pathologies et l'étiologie des neuropathies périphériques (NP) est vaste : troubles métaboliques, infections, toxines, blessures physiques et mutations génétiques, etc. La maladie de Charcot-Marie-Tooth 1A (CMT1A) est la neuropathie périphérique génétique héréditaire la plus fréquente. Elle est caractérisée par une surexpression de la protéine PMP22 impliquée dans le maintien de la gaine de myéline.

Actuellement, il n'existe pas de traitement pharmacologique pour cette affection. Il semble donc urgent de développer des molécules multi-cibles agissant simultanément sur différents aspects physiopathologiques.

### BREVE DESCRIPTION DE L'INVENTION

Récemment, l'intérêt pour le rôle des antioxydants alimentaires, tels que la curcumine, a suscité de nombreuses études. Cette molécule est depuis longtemps utilisée en médecine asiatique pour ses propriétés thérapeutiques. Cependant, elle n'est pas facilement absorbée au niveau intestinal et elle est vite dégradée en raison d'un métabolisme très rapide. Il en résulte que des doses très élevées de curcumine sont nécessaires pour obtenir des effets thérapeutiques sans avoir la certitude que la curcumine atteigne l'organe cible.

La curcumine est un polyphénol extrait de la racine de *Curcuma longa.* Cette molécule est depuis longtemps utilisée en médecine asiatique pour ses propriétés anti-inflammatoires et antibactériennes.

L'un des principaux obstacles à l'utilisation de la curcumine en thérapie est sa très faible biodisponibilité, ainsi que son métabolisme très rapide. La biodisponibilité d'une molécule s'entend comme la fraction de la dose administrée ou du principe actif libéré par la forme pharmaceutique qui parvient sous forme inchangée dans la circulation sanguine systémique et la vitesse à laquelle se réalise ce processus. Dans le cas d'une absorption par voie digestive, la curcumine est rapidement métabolisée via des mécanismes de conjugaison (en glucuronoconjugué et en sulfate). Ces modifications ont pour but de rendre la curcumine moins lipophile afin qu'elle soit éliminée plus rapidement par l'organisme. Le foie est l'organe principal au niveau duquel se déroulent ces réactions de conjugaison. De manière concomitante à la conjugaison et surtout dans le cas d'une administration intraveineuse, la curcumine subit des réactions par des enzymes hépatiques microsomales, les réductases. Sont ainsi formés des métabolites qui sont majoritairement des dérivés réduits de la curcumine : dihydrocurcumine, tétrahydrocurcumine, hexahydrocurcumine et hexahydrocurcuminol. Ces principaux métabolites subissent ensuite des réactions de conjugaison afin d'être éliminés rapidement ou excrétés.

Les présents inventeurs ont développé un composé innovant à base de nanocristaux de cellulose-cyclodextrine-curcumine (CNCs-CD-Cur ou Nano-Cur) afin de surmonter cet obstacle. Ce complexe permet d'améliorer considérablement la biodisponibilité de la curcumine et ainsi de diminuer la dose nécessaire pour produire des effets thérapeutiques, notamment sur les neuropathies périphériques.

Jusqu'à présent, la principale approche pharmacologique utilisée en clinique pour la prise en charge des neuropathies périphériques repose sur l'emploi d'analgésiques et d'anti-inflammatoires. Dans les situations où le nerf est partiellement ou complètement sectionné, des approches chirurgicales de suture ou de greffe de conduits nerveux, bio-fonctionnalisés ou non, sont utilisées. Néanmoins, l'efficacité de ces stratégies thérapeutiques reste limitée et ne s'attaque pas à la cause de la maladie, mais principalement aux symptômes. Cette absence de traitement pharmacologique est particulièrement dramatique dans le cas de neuropathies périphériques génétiques héréditaires, car celles-ci sont diffuses et se manifestent très précocement dans la vie des patients.

Les nanocristaux de cellulose-cyclodextrine-curcumine développés par les présents inventeurs constituent une nouvelle approche thérapeutique pour pallier la faible biodisponibilité de la curcumine et l'absence de traitement pharmacologique efficace des neuropathies périphériques.

### DESCRIPTION DETAILLEE DE L'INVENTION

Les maladies de Charcot-Marie-Tooth (CMT) sont les formes les plus courantes de neuropathies périphériques héréditaires motrices et sensitives. D'un point de vue épidémiologique, la prévalence globale des CMT est habituellement de 1/2500. Ces neuropathies résultent de la mutation de gènes codant des protéines responsables du maintien de la gaine de myéline et/ou des axones eux-mêmes. Les maladies de CMT sont un état caractérisé par un affaiblissement extrême et une atrophie des muscles des jambes et des pieds, des anomalies de la démarche, une perte des réflexes tendineux et un engourdissement des membres inférieurs. La forme CMT1A affecte environ 30 à 40% des patients atteints de la maladie de CMT. Dans cette neuropathie, il y a une perte et/ou une mauvaise formation de la gaine de myéline des fibres nerveuses périphériques, qui peut être caractérisée par une diminution de la vitesse de conduction nerveuse motrice des nerfs des membres supérieurs < 33-38 m/s. Les symptômes sensitifs sont habituellement moins importants et peuvent être subtils.

Il a été démontré dans de nombreuses études que la curcumine améliore la récupération fonctionnelle dans des modèles animaux de neuropathies héréditaires (Khajavi et al., 2005, Khajavi et al., 2007, Okamoto et al., 2013), auto-immunes (Han et al., 2014), alcooliques (Kandhare et al., 2012, Kaur et al., 2017), diabétiques (Lv et al., 2018, Daugherty et al., 2018), chimio-induites (Agthong et al., 2015, Al Moundhri et al., 2013) et traumatiques, y compris l'écrasement, la constriction chronique et la section complète du nerf (Liu et al., 2016; Mohammadi and Mahmoodi, 2013). Cependant, les doses utilisées dans ces études sont très élevées, entre 50 et 300 mg/kg/jour. Concernant la biodisponibilité de la curcumine, une première étude a été réalisée par Wahlstrom et Blennow en 1978, dans laquelle la curcumine a été administrée à des rats Sprague-Dawley à une dose de 1 g/kg. Dans cette étude, un faible taux de curcumine a été observé dans le plasma des rats. Ainsi, des études menées plus tard chez le rat ont permis de déterminer que la biodisponibilité orale de la curcumine est d'environ 1% (Shoba et al., 1998, Yang et al., 2007). Il en résulte que, chez l'Homme, des doses très élevées de curcumine (3,6 à 12 g) doivent être administrées, sans toutefois obtenir des concentrations plasmatiques de curcumine très élevées (Sharma et al., 2004). Il faut noter qu'aucun effet toxique n'a été rapporté chez l'Homme à ces doses (Sharma et al., 2004). Les propriétés bénéfiques de la curcumine sont entravées par sa faible solubilité (en raison de son hydrophobicité) et de sa faible biodisponibilité, combinées à une instabilité en solution qui entraîne une élimination rapide du sang (Sharma et al., 2004).

Dans une étude précédente, les inventeurs ont montré, dans un modèle de lésion traumatique du nerf sciatique chez le rat, qu'une faible dose de curcumine délivrée localement et en continu favorise la réparation nerveuse et la récupération fonctionnelle (Caillaud et al., 2018).

Des approches nano-technologiques ont été développées, notamment l'incorporation ou l'encapsulation de la curcumine dans des liposomes, des micelles polymériques, des nanoparticules polymériques, des nanogels, des nanoémulsions, des complexes d'inclusion, des nanoparticules lipidiques solides, des dendrimères, des phytosomes, des nanoparticules de silice mésoporeuse, ou des nanoparticules métalliques (Prasad et al., 2014). Tous ces nano-vecteurs ont tous permis d'augmenter la biodisponibilité et les effets bénéfiques de la curcumine. En revanche, aucun de ces nano-vecteurs associés à la curcumine n'a été testé sur les neuropathies périphériques. Les inventeurs ont développé des nano-biomatériaux capables de délivrer spécifiquement des composés naturels hydrophobes comme des dérivés chlorophylliens ou de la curcumine, mais également des siRNA. Ce nouveau système d'administration se compose de nanocristaux de cellulose (CNCs) qui bénéficient d'une bonne résistance mécanique, d'un caractère cristallin liquide, d'une surface spécifiquement élevée, d'une bonne biocompatibilité, biodégradabilité et durabilité. Obtenus par hydrolyse acide de fibres de coton, ces nanofibres sont définies comme des nanoparticules allongées de 100-200 nm de long, 10-20 nm de large et 5-10 nm d'épaisseur. Les charges négatives présentes à la surface des CNCs sont utilisées pour former des complexes ioniques avec des β-cyclodextrines (CD) cationiques, bien connues pour former des complexes d'inclusion avec des molécules hôtes. La β-CD est la plus couramment utilisée, en raison de sa synthèse relativement facile, de son faible coût et aussi du grand nombre de molécules polaires qui peuvent s'insérer dans sa cavité interne. Les CNCs ont été chargées de β-CD puis la curcumine a été incorporée dans la β-CD pour former des nanocristaux de curcumine- β-CD-CNCs (Ndong Ntoutoume et al., 2016). *In vitro,* ces nanocristaux de curcumine- β-CD-CNCs ont permis d'améliorer la pénétration intracellulaire de la curcumine. Cependant, les effets de ces nanocristaux n'avaient pas, jusqu'à présent, été testés *in vivo.*

La présente invention concerne un complexe comprenant :
- des nanocristaux de cellulose ;
- au moins une molécule de β-cyclodextrine ;
- au moins une molécule de curcumine,
pour une utilisation dans le traitement de tout type de neuropathies périphériques.

La synthèse du complexe sera détaillée ci-après en référence avec les figures annexées. En bref, les nanocristaux de cellulose ont été obtenus à partir d'une hydrolyse acide de la cellulose de fibres de cotons. La curcumine a été extraite à partir d'une poudre de curcuminoïdes et les β-cyclodextrines ont été fixées sur les nanocristaux de cellulose par réaction avec du chlorure de glycidyltriméthyl ammonium. Enfin, la curcumine a été incorporée dans les β-cyclodextrines afin d'obtenir le complexe de l'invention.

Dans un mode de réalisation, la neuropathie périphérique peut être une maladie de Charcot-Marie-Tooth. Les maladies de Charcot-Marie-Tooth (CMT) sont les formes les plus courantes de neuropathies périphériques héréditaires motrices et sensitives. Ces neuropathies résultent de la mutation de gènes codant des protéines responsables du maintien de la gaine de myéline et/ou des axones eux-mêmes. Dans un mode de réalisation particulier, la neuropathie périphérique peut être la maladie de Charcot-Marie-Tooth de type 1A. La maladie de Charcot-Marie-Tooth 1A (CMT1A) est la neuropathie périphérique génétique héréditaire la plus fréquente. Elle est caractérisée par une surexpression de la protéine PMP22 impliquée dans le maintien de la gaine de myéline.

Dans un autre mode de réalisation, la neuropathie périphérique peut être liée à une lésion traumatique.

En effet, la lésion d'un nerf périphérique peut être secondaire à un traumatisme ou une compression de ce nerf. Selon un aspect, le complexe est destiné à être administré à un sujet selon une quantité thérapeutiquement efficace. L'expression « quantité thérapeutiquement efficace » signifie le taux ou la quantité de composé nécessaire et suffisante pour ralentir ou stopper la progression, l'aggravation ou la détérioration d'un ou plusieurs symptômes de la neuropathie périphérique, en particulier la maladie de Charcot-Marie-Tooth ou une neuropathie périphérique d'origine traumatique et soulager les symptômes de la maladie.

La « quantité thérapeutiquement efficace » dépend du sujet, du stade de la maladie à traiter et du mode d'administration, et peut être déterminée par des opérations de routine par l'homme du métier. Cette quantité peut varier avec l'âge et le sexe du sujet.

En outre, la quantité thérapeutiquement efficace spécifique pour n'importe quel sujet dépendra d'une diversité de facteurs comprenant le trouble traité et la gravité du trouble ; l'activité du composé spécifique utilisé ; la composition spécifique utilisée, l'âge, la masse corporelle, l'état de santé général, le sexe et le régime du sujet ; la durée d'administration, la voie d'administration du composé spécifique utilisé ; la durée du traitement ; les médicaments utilisés en combinaison ou simultanément avec le composé spécifique utilisé ; et les facteurs similaires bien connus dans la technique médicale. Par exemple, il est bien dans les compétences de l'homme du métier de commencer des doses du composé à des taux inférieurs à ceux requis pour obtenir l'effet thérapeutique désiré et d'augmenter progressivement le dosage jusqu'à ce que l'effet désiré soit obtenu.

Dans un mode de réalisation particulier, la quantité thérapeutiquement efficace peut être comprise entre 0,02 et 200 mg/kg/jour de curcumine, en particulier entre 0,1 et 1 mg/kg/jour, plus particulièrement entre 0,1 et 0,3 mg/kg/jour.

Plus particulièrement, la quantité thérapeutiquement efficace peut être de 0,2 mg/kg/jour de curcumine.

Selon un autre aspect, le complexe est destiné à être administré à un sujet par voie injectable, de préférence sous-cutanée, intramusculaire, intraveineuse ou périneurale, par voie orale, par voie transdermique ou dans un conduit nerveux biofonctionnalisé implanté.

Des exemples de formes adaptées à l'injection comprennent, mais sans y être limitées, des solutions, telles que, par exemple, des solutions aqueuses stériles, des dispersions, des émulsions, des suspensions, des formes solides appropriées pour l'utilisation pour préparer des solutions ou suspensions par l'ajout d'un liquide avant l'utilisation, par exemple, une poudre, des formes liposomales ou similaires.

Le mode d'administration peut donc aussi être par injection ou par perfusion graduelle. L'injection peut être intraveineuse, intra-péritonéale, intramusculaire, sous-cutanée ou transdermale. Les préparations pour une administration parentérale peuvent inclure des solutions aqueuses ou non-aqueuses stériles, des suspensions ou des émulsions. Des exemples de solvants non-aqueux sont l'alcool benzylique, l'éthanol, le propylène glycol, le polyéthylène glycol, des huiles végétales ou des esters organiques injectables tels que l'éthyl oléate. Des véhicules aqueux comprennent l'eau, des solutions alcool/eau, des émulsions ou des suspensions.

Aussi, des exemples de formes adaptées pour l'administration par voie orale comprennent, mais sans y être limitées, des comprimés, des comprimés à orodispersion, des comprimés effervescents, des poudres, des granules, des pilules (comprenant des pilules édulcorées), des dragées, des gélules (comprenant des gélules de gélatine molle), des sirops, des liquides, des gels ou d'autres solutions, des suspensions, des bouillies, des formes liposomales et similaires.

Par ailleurs, on entend par « conduit nerveux biofonctionnalisé implanté » une prothèse nerveuse artificielle pour la réparation et la régénération de nerfs périphériques sous la forme de tubes de guidage. Ces tubes peuvent servir pour l'administration du complexe de la présente invention.

Dans un autre mode de réalisation, l'invention concerne une composition pharmaceutique comprenant :
- au moins le complexe de l'invention;
- au moins un excipient pharmaceutiquement acceptable,
pour une utilisation dans le traitement de tout type de neuropathies périphériques.

L'expression "excipient pharmaceutiquement acceptable" se réfère à un matériel non toxique qui est compatible avec un système biologique tel qu'une cellule, une culture cellulaire, un tissu ou un organisme. Cet excipient pharmaceutiquement acceptable ne produit pas de réaction indésirable, allergique ou autre lorsqu'il est administré à un animal, en particulier un être humain. Les caractéristiques de l'excipient dépendront du mode d'administration utilisé.

Ceci comprend n'importe quel solvant, diluant, milieu de dispersion, agglutinant, liant, lubrifiant, délitant, revêtement, agent antibactérien et antifongique, agent isotonique et agent retardateur d'absorption, et adjuvants similaires. Un excipient pharmaceutiquement acceptable se réfère à une charge solide, semi-solide ou liquide non-toxique, un diluant, une matière d'encapsulation ou une formulation accessoire de tout type. Pour l'administration humaine, les préparations devront satisfaire les exigences de stérilité, pyrogénicité, de sûreté générale et de pureté telles que requises par les Bonnes Pratiques de Fabrication des substances actives à usage humain et vétérinaire.

Dans un mode de réalisation particulier, la composition pharmaceutique est destinée à être administrée à un sujet selon une quantité thérapeutiquement efficace.

Dans la composition pharmaceutique de la présente invention, le complexe, seul ou en combinaison avec un excipient, peut être administré sous une forme d'administration unitaire, sous la forme d'un mélange avec des supports pharmaceutiques classiques, à des animaux et des êtres humains. Les formes d'administration unitaires appropriées comprennent les formes adaptées à la voie orale telles que les comprimés, les gélules, les poudres, les granules et les suspensions ou solutions pour voie orale, les formes d'administration sublinguale et buccale, les aérosols, les implants, les formes d'administration sous-cutanée, transdermique, topique, intrapéritonéale, intramusculaire, intraveineuse, sous-dermique, transdermique, intrathécale et intranasale et les formes d'administration rectale.

L'invention concerne l'utilisation du complexe ou de la composition pharmaceutique, sous la forme notamment d'hydrogel, d'implant sous-cutané, de pompe implantable, de conduit nerveux biofonctionnalisé implanté, pour améliorer l'observance du traitement, permettre une libération prolongée du complexe et obtenir une meilleure pharmacocinétique.

En effet, l'utilisation du complexe de la présente invention, par l'intermédiaire de l'amélioration considérable de la biodisponibilité de la curcumine dans sa forme complexée, permet une durée prolongée de libération du complexe comprenant la curcumine. Ceci contribue à faciliter l'observance du traitement par le sujet, traitement qui devient moins contraignant, avec des administrations plus espacées dans le temps en comparaison avec un traitement avec de la curcumine seule.

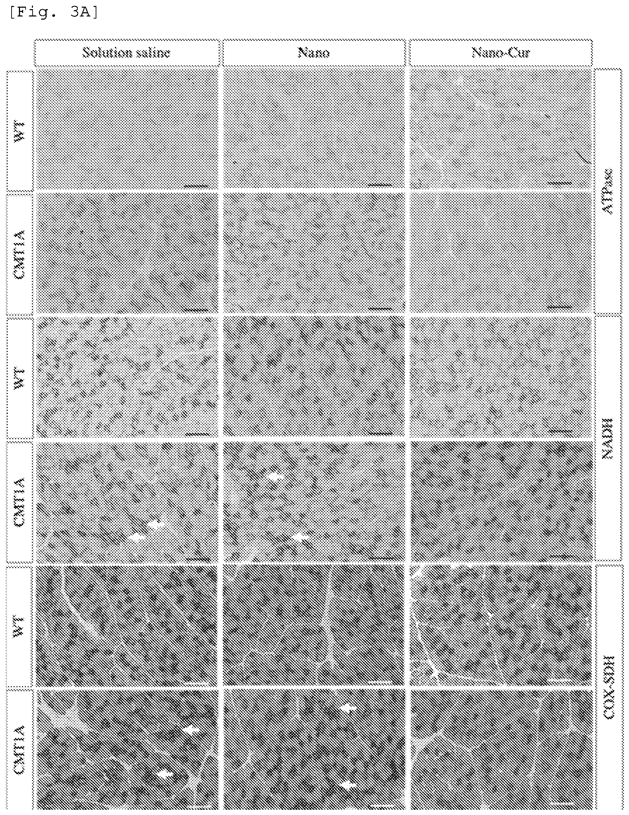

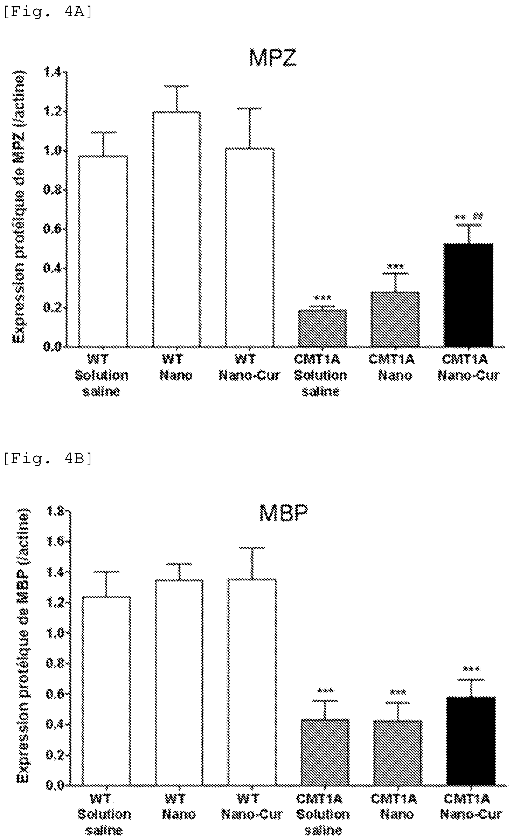

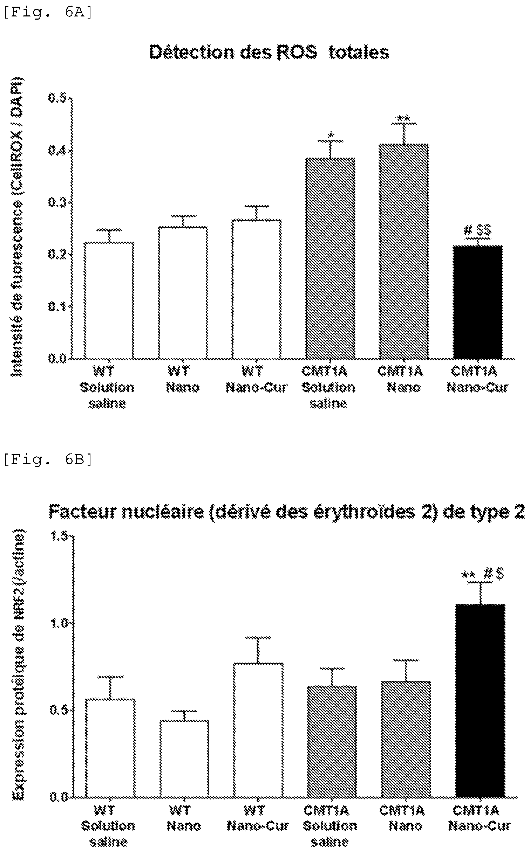

Pour mieux illustrer l'objet de la présente invention, on va maintenant en décrire ci-dessous, à titre illustratif et non limitatif, les exemples ci-après en liaison avec les dessins annexés :
[Fig. 1A] : schéma de la synthèse des nanocristaux de curcumine- β-cyclodextrine-cellulose (Nano-Cur).
[Fig. 1B] : imagerie à fluorescence *in vivo* de la dispersion des nanocristaux de cyclodextrine-cellulose (Nano) marqués par le fluorophore Dylight-800 chez le rat sauvage (WT) à différents temps post-injection.

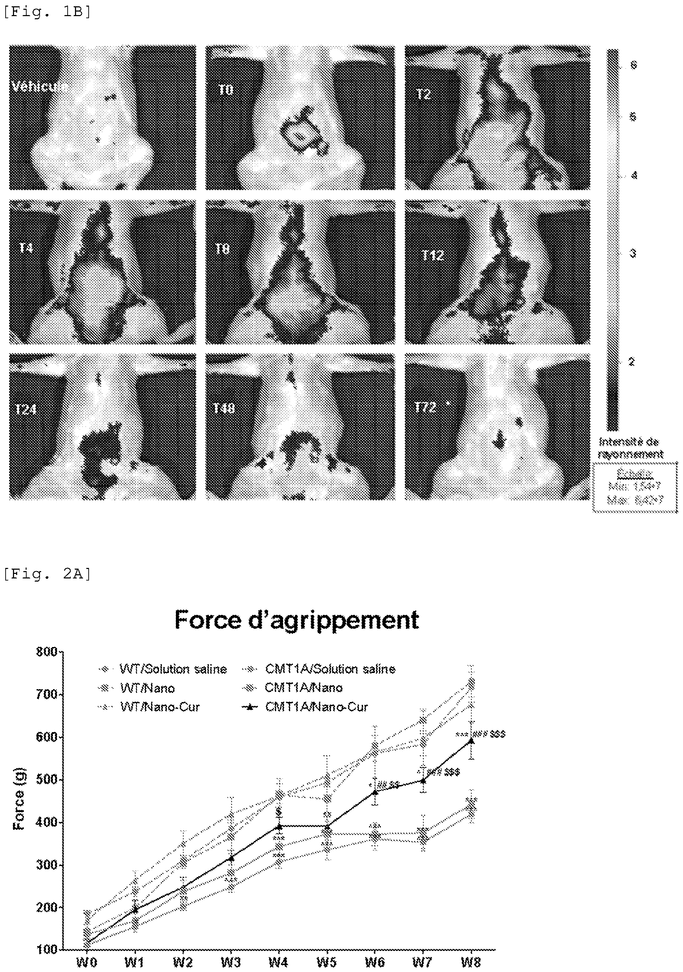

[Fig. 2A] : représentation graphique de la force d'agrippement des membres inférieurs de rats WT ou CMT1A en fonction de la durée du traitement par : soit une solution saline, soit les nanocristaux de cellulose-β-cyclodextrine dilués dans une solution saline (Nano), soit Nano-Cur (W : week = semaine). Les données sont exprimées sous forme de la moyenne +/- erreur type à l'aide d'une ANOVA à deux facteurs et d'un test post-hoc de Dunnett (* : p < 0,05, ** : p < 0,01 et *** : p < 0,001 par rapport au groupe WT/solution saline) et d'une ANOVA à deux facteurs que l'on fait suivre par un test post-hoc de Tukey (# : p < 0,05, ## : p < 0,01 et ### : p < 0,001 par rapport au groupe CMT1A/solution saline ; $ : p < 0,05, $$ : p < 0,01 et $$$ : p < 0,001 par rapport au groupe CMT1A/Nano).
[Fig. 2B] : représentation graphique de la performance d'équilibre (test sur barre) de rats WT ou CMT1A en fonction de la durée du traitement par : soit une solution saline, soit Nano, soit Nano-Cur.
[Fig. 2C] : représentation graphique du seuil de retrait à un stimulus mécanique de la patte (test des filaments de von Frey) de rats WT ou CMT1A en fonction de la durée du traitement par : soit une solution saline, soit Nano, soit Nano-Cur.
[Fig. 2D] : représentation graphique du seuil de retrait à un stimulus thermique de la patte (test de la plaque chauffante) de rats WT ou CMT1A en fonction de la durée du traitement par : soit une solution saline, soit Nano, soit Nano-Cur.
[Fig. 3A] : imagerie au microscope optique des colorations de coupes de muscles gastrocnémiens de rats WT ou CMT1A révélant i) l'ATPase pour évaluer la distribution des fibres musculaires squelettiques, ii) le NADH (nicotinamide adénine dinucléotide) pour évaluer le motif des types de fibres musculaires, iii) la cytochrome oxydase/succinique déshydrogénase (COX-SDH) pour montrer le motif des types de fibres musculaires, les fibres ayant des mitochondries anormales et les fibres dépourvues de cytochrome oxydase. Les flèches blanches indiquent la présence de regroupements (agrégats) de fibres musculaires. Barre d'échelle : 100 µm.
[Fig. 3B] : représentation graphique du dosage de créatine phosphokinase (CPK) en tant que marqueur de lyse musculaire.
[Fig. 3C] : représentation graphique du dosage de créatinine en tant que marqueur de lyse musculaire.
[Fig. 4A] : représentation graphique du résultat d'immunotransfert de MPZ (myelin protein zero) indiquant le taux d'expression de la protéine chez les différents groupes de rats testés. Les données sont comparées à l'aide d'une ANOVA à un facteur et d'un test post-hoc de Dunnett (* : p < 0,05, ** : p < 0,01 et *** : p < 0,001 par rapport au groupe WT/solution saline) et d'une ANOVA à un facteur que l'on fait suivre par un test post-hoc de Tukey (# : p < 0,05, ## : p < 0,01 et ### : p < 0,001 par rapport au groupe CMT1A/solution saline ; $ : p < 0,05, $$ : p < 0,01 et $$$ : p < 0,001 par rapport au groupe CMT1A/Nano).
[Fig. 4B] : représentation graphique du résultat d'immunotransfert de MBP (myelin basic protein) indiquant le taux d'expression de la protéine chez les différents groupes de rats testés.
[Fig. 4C] : représentation graphique du résultat d'immunotransfert de PMP22 (peripheral myelin protein 22) indiquant le taux d'expression de la protéine chez les différents groupes de rats testés.
[Fig. 4D] : immunohistochimie anti-PMP22 sur des coupes de nerfs sciatiques de rats WT ou de rats CMT1A. Barre d'échelle : 100 µm.
[Fig. 4E] : représentation graphique de la quantification d'ARNm PMP22 chez des rats de type sauvage WT ou des rats CMT1A.
[Fig. 5A] : représentation graphique de l'intensité de fluorescence de MBP (myelin basic protein) dans une co-culture de cellules de Schwann et neurones WT (contrôle) traitée ou non par Nano-Cur. Les résultats sont comparés à l'aide d'un test t (* : p < 0,05, ** : p < 0,01 et *** : p < 0,001 par rapport au contrôle).
[Fig. 5B] : représentation graphique de l'intensité de fluorescence de NFM (neurofilament) dans une co-culture de cellules de Schwann et neurones WT (contrôle) traitée ou non par Nano-Cur, permettant d'évaluer la quantité de neurites.
[Fig. 5C] : représentation graphique du rapport MBP/NFM dans une co-culture de cellules de Schwann et neurones WT traitée ou non par Nano-Cur, permettant d'évaluer la myélinisation.
[Fig. 6A] : représentation graphique de l'intensité de fluorescence comme mesure des espèces réactives de l'oxygène (ROS) totales à l'aide de la méthode cellROX dans des nerfs sciatiques de rats de type sauvage WT ou des rats CMT1A traités ou non par Nano-Cur. Les résultats sont comparés à l'aide d'une ANOVA à un facteur et d'un test post-hoc de Dunnett (* : p < 0,05, ** : p < 0,01 et *** : p < 0,001 par rapport au groupe WT/solution saline) et d'une ANOVA à un facteur que l'on fait suivre par un test post-hoc de Tukey (# : p < 0,05, ## : p < 0,01 et ### : p < 0,001 par rapport au groupe CMT1A/solution saline ; $ : p < 0,05, $$ : p < 0,01 et $$$ : p < 0,001 par rapport au groupe CMT1A/Nano).
[Fig. 6B] : représentation graphique du taux d'expression du facteur nucléaire Nrf2 (impliqué dans la production d'enzymes antioxydantes) chez des rats de type sauvage WT ou des rats CMT1A traités ou non par Nano-Cur.
[Fig. 6C] : représentation graphique du taux d'expression de la superoxyde dismutase [Cu-Zn] chez des rats de type sauvage WT ou des rats CMT1A traités ou non par Nano-Cur. Les résultats sont comparés à l'aide d'une ANOVA à un facteur et d'un test post-hoc de Tukey (* : p < 0,05, ** : p < 0,01 et *** : p < 0,001 par rapport au groupe WT/solution saline).
[Fig. 6D] : représentation graphique du taux d'expression de la glutathione S-transférase alpha-3 chez des rats de type sauvage WT ou des rats CMT1A traités ou non par Nano-Cur.
[Fig. 7A] : représentation graphique de l'intensité de fluorescence comme mesure des espèces réactives de l'oxygène (ROS) totales à l'aide de la méthode cellROX sur des cellules de Schwann de type sauvage soumises à un stress oxydant induit par H₂O₂, en fonction de diverses doses de curcumine seule ou de Nano-Cur. Les résultats sont comparés à l'aide d'une ANOVA à un facteur et d'un test post-hoc de Tukey (* : p < 0,05, ** : p < 0,01 et *** : p < 0,001 par rapport au groupe contrôle ; § : p < 0,05, §§ : p < 0,01 et §§§ : p < 0,001 par rapport au groupe contrôle + curcumine).
[Fig. 7B] : représentation graphique de l'intensité de fluorescence comme mesure de la production de superoxydes par les mitochondries à l'aide de la méthode MitoSOX sur des cellules de Schwann de type sauvage soumises à un stress oxydant induit par H₂O₂, en fonction de diverses doses de curcumine seule ou de Nano-Cur.
[Fig. 7C] : représentation graphique de l'intensité de fluorescence comme mesure du potentiel membranaire mitochondrial à l'aide la méthode Rho123.

### EXEMPLES

Les exemples suivants illustrent l'invention.

### Matériels et Méthodes

### Synthèse des nanocristaux de cellulose-cyclodextrine-curcumine (CNCs-CD-Cur ou Nano-Cur)

Une hydrolyse acide (H₂SO₄) de fibres de coton a permis d'obtenir des nanocristaux de cellulose (CNCs) chargés négativement. La cyclodextrine cationique (CD) a été préparée par réaction de la β-cyclodextrine (β-CD) avec du chlorure de glycidyltriméthylammonium puis a été fixée par interaction ionique sur les CNCs afin de former le complexe CNCs-CD (Nano). En parallèle, la curcumine a été extraite à partir d'une poudre de curcuminoïdes (curcuma) puis a été encapsulée dans les CD afin d'obtenir les complexes curcumine-CD-CNCs (CNCs-CD-Cur ou Nano-Cur).

### Animaux

Les expériences ont été réalisées sur 60 rats mâles âgés d'un mois (30 rats hétérozygotes CMT1A et 30 rats Sprague Dawley de type sauvage). La génération des rats CMT1A (PMP22 transgéniques) est décrite dans Sereda et al. (1996).

Les protocoles expérimentaux ont été spécifiquement approuvés par le Comité régional d'éthique en expérimentation animale (CREEAL n°16-2013-16, APAFIS#18160-2018122015432214 v1).

### Traitement des rats

Des rats transgéniques CMT1A (mâles âgés d'un mois) ont été traités par les nanocristaux de cellulose-cyclodextrine-curcumine (Nano-Cur) quotidiennement par une injection intrapéritonéale à raison de 0,2 mg/kg/jour de curcumine pendant 8 semaines.

Les rats ont été aléatoirement répartis en 6 groupes : rats de type sauvage WT traités par une solution saline (WT/solution saline, n=10), rats WT traités par des nanocristaux de cellulose-cyclodextrine (Nano) solubilisés dans une solution saline (WT/Nano, n=10), rats WT traités par Nano-Cur solubilisés dans une solution saline (WT/Nano-Cur, n=10), rats CMT1A traités par une solution saline (CMT1A/solution saline, n=10), rats CMT1A traités par Nano solubilisés dans une solution saline (CMT1A/Nano, n=10) et rats CMT1A traités par Nano-Cur solubilisés dans une solution saline (CMT1A/Nano-Cur, n=10).

### Tests comportementaux

### Sensibilité thermique

Les latences de retrait suite à un stimulus thermique ont été mesurées à l'aide d'une plaque chaude de 52°C (Bioseb, France) de façon hebdomadaire pendant 8 semaines. Après 10 minutes d'acclimatation, les animaux ont été placés sur la plaque chauffante jusqu'à l'expression de comportements nocicepteurs tels que le tremblement des pattes arrière ou le saut des animaux. La durée maximale du test a été fixée à 30 secondes afin d'éviter les dommages des tissus. Trois tests séparés chacun de 10 minutes ont été réalisés et la valeur moyenne a été utilisée pour représenter le seuil de nociception thermique pour chaque animal.

### Sensibilité mécanique

Le seuil de retrait des pattes arrières en réponse à un stimulus mécanique a été mesuré de façon hebdomadaire, pendant 8 semaines, à l'aide d'une série de filaments de von Frey (Bio-VF-M, Bioseb, France).

### Test d'agrippement

Des tests de force d'agrippement ont été réalisés de façon hebdomadaire pendant 8 semaines avec la barre en T d'un dispositif de mesure de force d'agrippement (BIO-GS3, Bioseb, France).

### Test d'équilibre

Le test de maintien sur une barre surélevée a permis d'évaluer la force musculaire des quatre pattes et les performances d'équilibre des animaux. Ce test a été réalisé de façon hebdomadaire pendant 8 semaines. Le rat était placé sur ses quatre pattes au milieu de la barre en bois (diamètre : 2,5 cm, longueur : 50 cm, hauteur 30 cm). La durée passée sur la barre (latence jusqu'à la chute) dans chaque essai a été enregistrée. Trois tests séparés de 10 minutes ont été réalisés et la valeur moyenne de ces tests a été calculée pour représenter la performance d'équilibre.

### Analyse électrophysiologique

L'onde M et le réflexe H ont été mesurés avec le PowerLab/26T (ADInstruments, France) après la stimulation supramaximale des régions distale et proximale du nerf sciatique. La vitesse de la conduction nerveuse motrice (MNCV : motor nerve conduction velocity) et la vitesse de conduction nerveuse sensitive (SNCV : sensitive nerve conduction velocity) ont été calculées respectivement à partir de la latence de l'onde M et de celle du réflexe H.

### Microscopie optique et analyse morphométrique

Des échantillons de nerf sciatique ont été fixés dans 2,5% de glutaraldéhyde et inclus dans une résine époxy (Euromedex, France). Des coupes transversales semi-fines (1,5 µm) ont été marquées au bleu de toluidine et utilisées pour des évaluations morphométriques, qui ont été réalisées en simple aveugle. Les dommages tissulaires ont été estimés par le nombre total d'axones myélinisés par section de nerf et le diamètre moyen des fibres nerveuses. L'épaisseur de la gaine de myéline a été calculée par la formule :
(diamètre de la fibre - diamètre de l'axone)/2. Le degré de myélinisation (G-ratio) a été estimé par le ratio du diamètre de l'axone à celui de la fibre et le ratio de l'épaisseur de myéline au diamètre de l'axone.

### Immunohistochimie

Des échantillons de nerf sciatique ont été inclus dans une colle OCT et congelés dans l'azote liquide. Des coupes au cryostat (5 µm) ont été séchées pendant 4 heures sur des lames Superfrost/+, puis par une fixation avec 4% de paraformaldéhyde pendant 10 minutes à température ambiante a été effectuée. La perméabilisation des coupes a été réalisée par incubation dans 0,1% Tween20 dans du PBS pendant 15 minutes. La liaison non spécifique a été bloquée à l'aide de 10% de BSA dans du PBS pendant 1 heure. L'anticorps primaire anti-PMP22 (SAB4502217-100UG, Sigma Aldrich, 1:200) a été ajouté dans 4% BSA dans du PBS, pendant une nuit à 4°C. Les échantillons ont ensuite été rincés pendant 15 minutes dans du PBS à température ambiante, puis incubés avec l'anticorps secondaire (anti-lapin fabriqué chez l'âne Alexa-fluor 594, Dako, 1:200) dans 4% BSA dans du PBS pendant 2 heures à température ambiante. Les lamelles ont été montées à l'aide du milieu de montage en fluorescence de Dako (Dako France S.A.S, France). La fluorescence rouge a été visualisée et l'intensité mesurée par microscopie à fluorescence (microscope optique Nikon H600L) et les images ont été capturées à l'aide d'un appareil photo numérique Nikon (Nikon, France).

### Western blot

Les protéines ont été séparées par électrophorèse SDS-PAGE puis transférées sur une membrane de cellulose. Les blots ont été incubés avec des anticorps primaires monoclonaux de souris reconnaissant soit l'actine β-cytoplasmique, la myelin protein zero (MPZ), la myelin basic protein (MBP) ou avec des anticorps polyclonaux de lapin reconnaissant la peripheral myelin protein 22 (PMP22) ou le facteur nucléaire (dérivé d'érythroïde) de type 2 (Nrf2).

### Histoenzymologie du muscle gastrocnémien

Des fragments de muscle gastrocnémien ont été inclus dans une colle OCT, congelés dans de l'azote liquide et stockés à -80°C. Des coupes transversales de muscle (5 µm) ont été réalisées à l'aide d'un cryotome UV Leica CM 1850 à 25°C et collectées sur des lames en verre. Les coupes ont ensuite été colorées à l'aide soit de la réaction de la m-ATPase avec une pré-incubation alcaline et acide, de la méthode de Padykula et Herman (1955) ; de la réaction de NADH-TR selon la méthode de Pearse (1968) modifiée par Dobowitz et Brooke (1973) ou de la réaction de la succinate déshydrogénase (COX-SDH) décrite par Nachalas et al. (1957) et modifiée par Wegman et Tordet-Coidroit (1960). Pour chaque échantillon, des images ont été acquises à l'aide d'un microscope optique Nikon H600L (Nikon, Japon) et ont été prises à partir de 3 champs choisis aléatoirement.

### Détection des espèces réactives de l'oxygène dans les nerfs sciatiques

Des coupes transversales de nerfs sciatiques réalisées au cryostat (5 µm) ont été lavées avec du PBS et incubées avec 5µM de réactif CellROX-Green (Life Technologies GmbH, Allemagne) pendant 30 minutes à 37°C. Les noyaux ont été colorés à l'aide de DAPI. Les espèces réactives de l'oxygène (ROS) ont été évaluées par intensité de fluorescence à l'aide d'un microscope à fluorescence.

### Culture cellulaire et évaluation du stress oxydant in vitro Culture de cellules de Schwann

Les cellules de Schwann (SC) ont été isolées à partir de nerfs sciatiques de rats mâles sauvages (WT) ainsi que de rats CMT1A âgés de 8 semaines. Les SC ont été mises en culture dans un milieu de culture de SC (décrit dans Caillaud et al., 2018) : DMEM D-valine + 2mM de glutamine (Gibco) + 10% de sérum de veau fœtal (Gibco) + 1% de supplément N2 (Gibco) + 20µg/mL d'extrait pituitaire bovin + 5 µM de forskoline (Gibco) + 100 U/mL de pénicilline/streptomycine (Gibco) + 250 µg/mL de fungizone (Gibco). Les SC ont été identifiées l'aide d'un marquage immunofluorescent pour la protéine S-100.

### Stress oxydant in vitro

Les SC de type sauvage ont été prétraitées par la curcumine (0,001, 0,01, 0,1, 1 ou 10 µM) (Sigma) ou par Nano-Cur (0,001, 0,01, 0,1, 1 ou 10 µM) pendant 8 heures. Pour induire un stress oxydant, les SC ont été soumises à un traitement par H₂O₂ (0,1 mM) pendant 8 heures. Les ROS totales et les ions superoxydes mitochondriaux ont été évalués à l'aide du réactif CellROX-Green (Life Technologies GmbH) et l'indicateur de superoxyde mitochondrial MitoSOX Red (Life Technologies GmbH) respectivement, selon les recommandations du fabricant. De plus, les effets de la curcumine et de Nano-Cur sur la production de ROS ont été évalués par la détermination du potentiel membranaire mitochondrial (Δψm), qui a été réalisée par incubation avec de la rhodamine 123 (Rho123), tel que décrit précédemment dans Caillaud et al. (2018).

### Myélinisation dans des co-cultures de cellules de Schwann et de neurones

Des co-cultures de neurones sensoriels (WT) et de cellules de Schwann (CMT1A) ont été établies selon des procédés déjà publiés (Nobbio et al., 2004). En bref, les co-cultures ont été maintenues pendant 30 jours dans un milieu neurobasal (Invitrogen, Srl) supplémenté par 15% de sérum de veau foetal (NCS, Invitrogen, Srl) et 5 ng/mL de NGF (Invitrogen, Srl). Les co-cultures ont été traitées pendant 1 semaine avec 0,01 µM de Nano-Cur. Pour évaluer les changements de morphologie cellulaire et la myélinisation, des marquages immunofluorescents de MBP (myelin basic protein) et de NFM (protéine M des neurofilaments) ont été réalisés.

### Analyse statistique

Toutes les données sont exprimées en tant que moyenne +/erreur-type. Si la normalité (test de Shapiro-Wilk) et l'homogénéité des variances (test de Brown-Forsythe) ont été observées, alors les données ont été comparées à l'aide d'une ANOVA soit à un facteur soit à deux facteurs avec des mesures répétées, que l'on fait suivre par un test de comparaison *post-hoc* de Tukey ou de Dunnett. Si la normalité et l'homogénéité des variances n'étaient pas observées, alors les données ont été comparées à l'aide d'un test de Krustal-Wallis suivi par un test *post-hoc* de Dunn. Toutes les analyses statistiques ont été réalisées avec un logiciel statistique Graphpad (GraphPad Software, Inc., USA). Les différences ont été considérées significatives quand p était <0,05, <0,01 ou <0,001.

### Exemple 1 : les nanocristaux CNCs-CD-Curcumine (Nano-Cur) améliorent la biodisponibilité de la curcumine in vivo

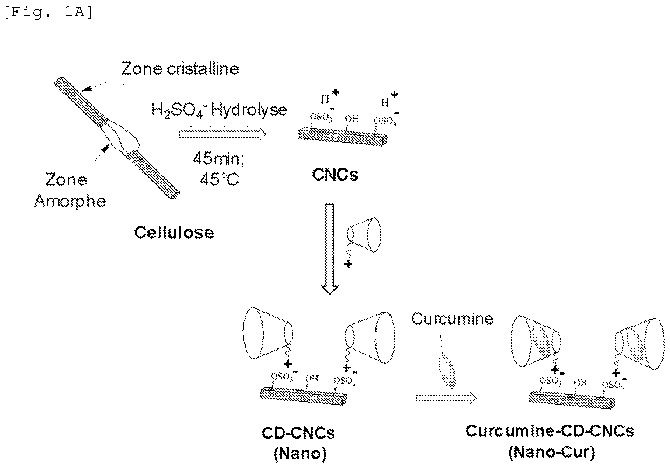

Si l'on se réfère à la Figure 1A, après hydrolyse acide (H₂SO₄) de fibres de coton, les nanocristaux de cellulose (CNCs) obtenus présentent des charges de surfaces négatives, leur conférant une stabilité en solution aqueuse. Ces CNCs sont ensuite fonctionnalisés par des cyclodextrines cationiques (CD) obtenues par réaction avec le chlorure de glycidyltriméthylammmonium et la β cyclodextrine. Enfin, la curcumine est encapsulée dans les β CD du complexe CNCs-CD (Nano).

L'injection des nanocristaux CNCs-CD seuls (Nano) marqués par le fluorophore Dylight 800 a montré, au moyen de l'imagerie *in vivo,* une dispersion des nanocristaux CNCs-CD dans l'ensemble de l'organisme de l'animal (Figure 1B) et que celles-ci étaient détectables jusqu'à 72 heures après l'injection.

Par ailleurs, les résultats présentés dans le Tableau 1 ci-après ont montré que les nanocristaux de curcumine (CNCs-CD-Curcumine ou Nano-Cur) ont permis d'augmenter la biodisponibilité de la curcumine de 250 fois par comparaison à l'administration de curcumine seule, et de maintenir une libération prolongée de la curcumine dans le plasma (au moins 12 heures après administration).

**Tableau 1 : Biodisponibilité de la curcumine dans le plasma**

| [Tableau 1] | | | | | | |
|---|---|---|---|---|---|---|
| | Curcumine 50 mg/kg | | | Nano-Cur 0,2 mg/kg | | |
| Temps (heures) | Concentration (ng/mL) | SEM | Nombre | Concentration (ng/mL) | SEM | Nombre |
| 0 | 0 | ±0 | n=3 | 0 | ±0 | n=3 |
| 1 | 24,9 | ±0,2 | n=3 | 26,3 | ±16,1 | n=3 |
| 2 | 4,3 | ±0,7 | n=3 | 5,3 | ±0,7 | n=3 |
| 8 | 0 | ±0 | n=3 | 15,4 | ±8,9 | n=3 |
| 12 | 0 | ±0 | n=3 | 10,8 | ±6,5 | n=3 |

### Exemple 2 : Le traitement par les nanocristaux de CNCs-CD-Curcumine (Nano-Cur) améliore le phénotype des rats CMT1A

Les analyses réalisées à partir des données des tests comportementaux dans tous les groupes de type sauvage (WT) n'ont indiqué aucune différence significative entre les groupes (WT/solution saline, WT/Nano et WT/Nano-Cur) pendant les 8 semaines de tests. De plus, aucune différence significative n'a été observée entre les groupes CMT1A/solution saline et CMT1A/Nano.

La Figure 2A montre la performance d'agrippement des pattes arrières des animaux mesurée à l'aide du test de force d'agrippement. Une diminution significative de la force d'agrippement a été révélée chez les rats CMT1A/solution saline par comparaison aux rats WT/solution saline aux semaines W1 (p < 0,05), W2, W3 (p < 0,01), W4, W5, W6, W7 et W8 (p < 0,001). D'une manière similaire, une perte de performance de force d'agrippement a été observée chez les rats CMT1A/Nano par comparaison aux rats WT/solution saline aux semaines W4 (p < 0,01), W5, W6, W7 et W8 (p < 0,001). D'une manière intéressante, les analyses ont montré une amélioration significative de la force d'agrippement chez les rats CMT1A/Nano-Cur par comparaison aux rats CMT1A/solution saline aux semaines W4 (p < 0,05), W6 (p < 0,01), W7 et W8 (p < 0,001). De manière similaire, une augmentation de la force d'agrippement a été observée chez les rats CMT1A/Nano-Cur par comparaison aux rats CMT1A/Nano aux semaines W6 (p < 0,001), W7 (p < 0,001) et W8 (p < 0,001). Ces résultats suggèrent donc que le traitement Nano-Cur limite la perte de force d'agrippement observée chez les rats CMT1A.

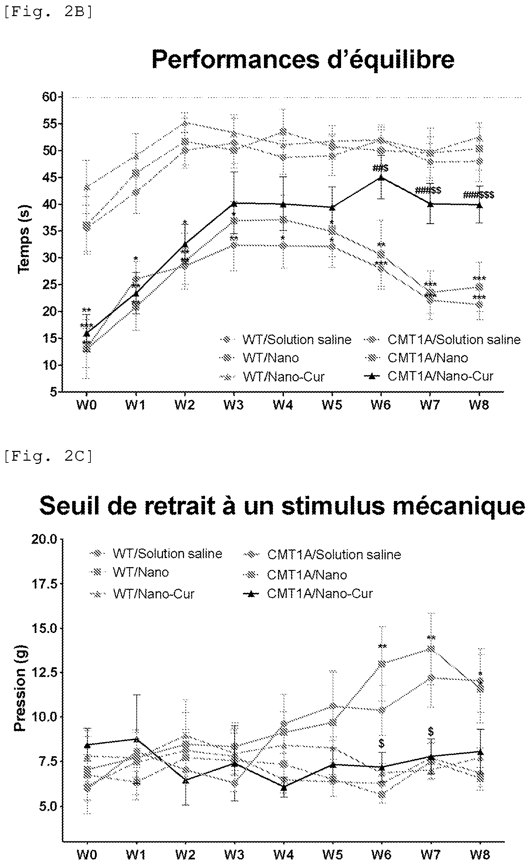

L'équilibre des animaux a été évalué à l'aide du test de maintien sur une barre (Figure 2B). Une diminution significative de la durée d'équilibre a été observée chez les rats CMT1A/solution saline par comparaison aux rats WT/solution saline à W0, W1, W2, W3 (p < 0,01), W4, W5 (p < 0,05), W6, W7 et W8 (p < 0,001). De façon similaire, une diminution significative de la performance d'équilibre a été observée chez les rats CMT1A/Nano par comparaison aux rats WT/Nano à W0 (p < 0,001), W1, W2 (p < 0,01), W3, W5 (p < 0,05), W6, W7 et W8 (p < 0,001). Les analyses statistiques réalisées à partir des données des rats CMT1A/Nano-Cur ont indiqué une diminution significative de la performance d'équilibre sur les trois premières semaines de traitement par comparaison aux animaux CMT1A/solution saline (W0 : p < 0,01, W1 : p < 0,05 et W2 : p < 0,05). Cependant, aucune différence significative n'a été observée entre les groupes CMT1A/Nano-Cur et WT/solution saline pendant les six dernières semaines de traitement. De manière intéressante, une amélioration significative de la performance d'équilibre a été notée chez les rats CMT1A/Nano-Cur par comparaison aux rats CMT1A/solution saline (W6 : p < 0,01, W7 et W8 : p < 0,001) et les rats CMT1A/Nano (W6 : p < 0,05, W7 : p < 0,01 et W8 : p < 0,001) aux semaines W6, W7 et W8. Ces résultats suggèrent un effet bénéfique du traitement par Nano-Cur sur la performance d'équilibre des rats CMT1A.

La réponse à un stimulus mécanique a été évaluée à l'aide du test des filaments de von Frey (Figure 2C). A la semaine W8, une augmentation significative de la pression provoquant un retrait de la patte a été observée chez les animaux CMT1A/solution saline par comparaison aux animaux WT/solution saline (W8 : p < 0,05) et une tendance en ce sens aux semaines W6 et W7. Aussi, à W6, W7 et W8, une diminution significative de la sensibilité tactile a été observée chez les rats CMT1A/Nano par comparaison aux rats WT/solution saline (W6 : p < 0,01 ; W7 : p < 0,01 et W8 : p < 0,05). De manière intéressante, une amélioration significative de la sensibilité tactile a été montrée chez les animaux CMT1A/Nano-Cur à W6 (p < 0,05) et W7 (p < 0,05) par comparaison aux rats CMT1A/Nano. En outre, une analyse statistique n'a indiqué aucune différence significative pendant les 8 semaines de test entre les groupes CMT1A/Nano-Cur et WT/solution saline. Ces résultats suggèrent que le traitement par Nano-Cur limite la perte de sensibilité tactile chez les rats CMT1A.

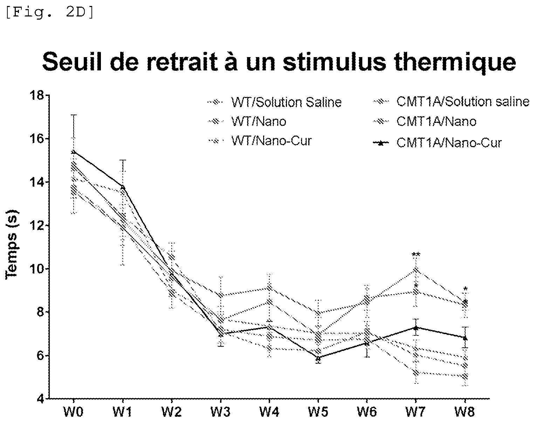

La réponse à un stimulus thermique a été évaluée à l'aide du test de la plaque chaude (Figure 2D). Aux semaines W7 et W8, une augmentation significative de la latence de retrait de la patte a été observée chez les rats CMT1A/solution saline et CMT1A/Nano par comparaison aux rats WT/solution saline (CMT1A/solution saline : W7 p < 0,01 et W8 p < 0,05, CMT1A/Nano : W7 p < 0,05 et W8 p < 0,05). En revanche, aucune différence significative dans la sensibilité thermique n'a été observée entre les rats WT/solution saline et CMT1A/Nano-Cur pendant les 8 semaines de traitement. Ces résultats suggèrent que le traitement par Nano-Cur améliore la sensibilité thermique des rats CMT1A.

Une analyse électromyographique a été réalisée à la semaine 8 (Tableau 2). Des analyses statistiques ont été effectuées à partir des données de vitesse de conduction nerveuse motrice (NMCV) et de l'amplitude de l'onde M : elles n'ont indiqué aucune différence significative entre tous les groupes WT. En revanche, une diminution de la NMCV et de l'amplitude de l'onde M a été observée chez tous les groupes CMT1A. Néanmoins, une augmentation de la NMCV et de l'amplitude des ondes M a été observée chez les rats CMT1A/Nano-Cur par comparaison aux rats CMT1A/solution saline (p < 0,001) ou aux rats CMT1A/Nano (p < 0,001). Les analyses des données de vitesse de conduction nerveuse sensitive (SNCV) et de l'amplitude du réflexe H n'ont pas indiqué de différences significatives entre tous les groupes WT. Chez les rats CMT1A/Nano-Cur, il a été possible d'enregistrer le réflexe H (contrairement aux deux autres groupes de rats CMT1A), permettant ainsi de calculer une SNCV, laquelle est néanmoins restée moindre que celle des rats WT/solution saline (amplitude de réflexe H : p-<-0,001, SNCV : p-<-0,001). Ces résultats suggèrent que le traitement par Nano-Cur limite la diminution des paramètres électrophysiologiques observée chez les rats CMT1A.

### Tableau 2 : Enregistrements électrophysiologiques

**[Tableau 2]**

| | | | | | | |
|---|---|---|---|---|---|---|
| | WT/solution saline | WT/Nano | WT/ Nano-Cur | CMT1A/solution saline | CMT1A/Nano | CMT1A/Nano-Cur |
| MNCV (m/s) | 39,9 (±4,7) | 41,5 (±5,0) | 39,5 (±3,8 ) | 14,1 (±1,2)** * | 14,9 (±1,3)** * | 25,0 (±2,4)** ### $$$ |
| Amplitud e de l'onde M (mV) | 15,7 (±0,9) | 14,9 (±0,9) | 14,8 (±1,2 ) | 2,5 (±0,5)** * | 2,3 (±0,5)** * | 7,2 (±0,7)** * ### $$$ |
| SNCV (m/s) | 35,9 (±3,9) | 37,4 (±3,8) | 37,4 (±3,8 ) | N.D. | N.D. | 12,5 (±1,7)** * |
| Amplitud e du réflexe H | 1,6 (±0,3) | 1,7 (±0,3) | 1,6 (±0,2 ) | N.D. | N.D. | 0,4 (±0,1)** * |

Les valeurs d'électrophysiologie sont exprimées en tant que moyenne ± erreur-type (SEM). Les résultats sont comparés à l'aide d'une ANOVA à un facteur et un test *post-hoc* de Dunnett (* : p<0,05, ** : p<0,01 et *** : p<0,001 par rapport au groupe WT/solution saline) et à l'aide d'une ANOVA à un facteur que l'on fait suivre par un test *post-hoc* de Tukey (# : p<0,05, ## : p<0,01 et ### : p<0,001 par rapport au groupe CMT1A/solution saline ; $ : p<0,05, $$ : p<0,01 et $$$ : p<0,001 par rapport au groupe CMT1A/Nano).

### Exemple 3 : Le traitement par Nano-Cur limite les dommages musculaires chez les rats CMT1A

La coloration de l'ATPase a été utilisée pour évaluer la distribution des fibres musculaires squelettiques. La coloration combinée de la cytochrome oxydase et de l'acide succinique déshydrogénase (COX-SDH) a été utilisée pour montrer le type de fibres musculaires, les fibres avec des mitochondries anormales et les fibres dépourvues de cytochrome oxydase. La coloration au NADH a été utilisée pour évaluer le type de fibres musculaires, la distribution des mitochondries et la perturbation des myofibrilles. L'analyse microscopique de ces différentes colorations histochimiques n'a montré aucune différence dans la dimension des fibres musculaires du muscle gastrocnémien ou dans la distribution des mitochondries dans tous les groupes de rats à W8.

Le regroupement des fibres musculaires de type I et/ou de type II est un signe précoce de dommage musculaire. Si l'on se réfère à la Figure 3A, une tendance au regroupement des fibres musculaires a été observée chez les rats CMT1A/solution saline et CMT1A/Nano par comparaison aux rats WT/solution saline. Cependant, ce regroupement des fibres musculaires n'a pas été observé chez les rats CMT1A/Nano-Cur.

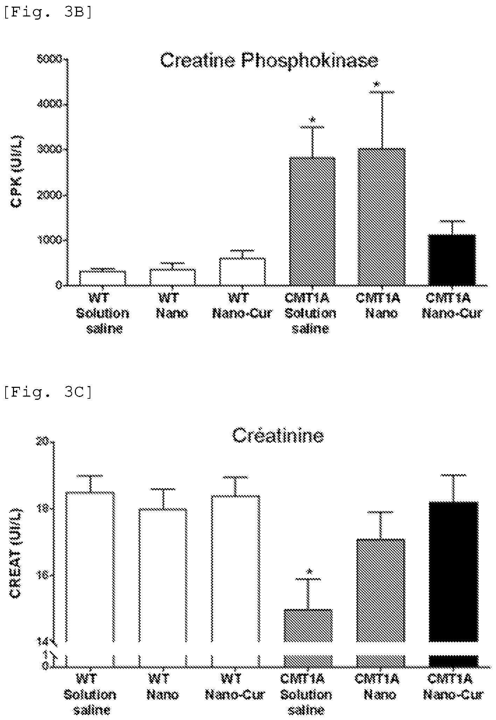

De plus, les dosages plasmatiques de marqueurs de lyse musculaire (créatine phosphokinase CPK et créatinine) ont montré une augmentation de la CPK (p < 0,05) (Figure 3B) et une diminution de la créatinine (p < 0,05) (Figure 3C) chez les rats CMT1A/solution saline par comparaison au groupe WT/solution saline. Aucune différence significative n'a été observée dans les taux de CPK et de créatinine chez les rats CMT1A/Nano-Cur par rapport aux rats WT/solution saline. Ces résultats indiquent un effet bénéfique du traitement par Nano-Cur sur l'intégrité du tissu musculaire gastrocnémien chez les rats CMT1A.

### Exemple 4 : Le traitement par Nano-Cur limite la démyélinisation in vivo et améliore la myélinisation in vitro

Des analyses morphométriques ont été réalisées à l'aide d'images microscopiques obtenues à partir de coupes semi-fines (W8). Aucune différence significative n'a été identifiée dans les paramètres morphométriques entre tous les groupes WT. De plus, aucune différence significative n'a été observée dans le nombre d'axones myélinisés entre tous les groupes (Tableau 3). Par comparaison aux rats WT/solution saline, le diamètre moyen des axones et le diamètre moyen des fibres (axone + myéline) ont été réduits de manière significative dans tous les groupes CMT1A (p < 0,001). Cependant, une augmentation significative du diamètre des fibres myélinisées a été observée chez le groupe CMT1A/NanoCur par rapport au groupe CMT1A/Nano (p<0,05). Une diminution significative de l'épaisseur de la myéline a été observée dans tous les groupes CMT1A (p<0,001) par comparaison au groupe WT/solution saline. En revanche, une augmentation significative de l'épaisseur de la myéline chez les rats CMT1A/Nano-Cur a été mise en évidence par rapport aux rats CMT1A/solution saline (p < 0,05) ou aux rats CMT1A/Nano (p < 0,05).

De manière similaire, une diminution du G-ratio a été observée dans tous les groupes CMT1A (p < 0,001) par comparaison au groupe WT/solution saline. Néanmoins, une augmentation significative du G-ratio a été observée chez les rats CMT1A/Nano-Cur par comparaison au groupe CMT1A/solution saline (p < 0,05) et également une augmentation significative de l'épaisseur de myéline par comparaison aux animaux CMT1A/solution saline (p < 0,05) ou CMT1A/Nano (p < 0,05).

**Tableau 3 : Caractéristiques des axones myélinisés : nombre total, diamètre moyen des axones et diamètre moyen des fibres (axone + myéline), G-ratio et épaisseur de la myéline**

| [Tableau 3] | | | | | | |
|---|---|---|---|---|---|---|
| | WT/ solution saline | WT/Nano | WT/ Nano-Cur | CMT1A/ solution saline | CMT1A/ Nano | CMT1A/ Nano-Cur |
| Nombre d'axones | 1796 (±62,1) | 1766 (±71,5) | 1741 (±58,1) | 1636 (±44,2) | 1616 (±58,6) | 1679 (±64,1) |
| Diamètre d'axone | 5,7 (±0,2) | 5,6 (±0,2) | 5,6 (±0,2) | 4,3 (±0,1)*** | 4,1 (±0,2)*** | 4,4 (±0,1) * ** |
| Diamètre de fibre | 9,8 (±0,3) | 9,6 (±0,2) | 9,9 (±0,3) | 5,9 (±0,2)*** | 5,8 (±0,2)*** | 6,7 (±0,3)* **$ |
| G-ratio | 0,57 (±0,01) | 0,57 (±0,01) | 0,56 (±0,01) | 0,71 (±0,01)*** | 0,69 (±0,02)*** | 0,64 (±0,01) ** # |
| Epaisseur de la myéline | 2,05 (±0,08) | 2,00 (±0,04) | 2,12 (±0,07) | 0,88 (±0,02)*** | 0,86 (±0,03)*** | 1,18 (±0,10) *** #$ |

| | | | | | | |
|---|---|---|---|---|---|---|
| Les résultats sont comparés à l'aide d'une ANOVA à un facteur et d'un test *post-hoc* de Dunnett (* : p<0,05, ** : p<0,01 et *** : p<0,001 par rapport au groupe WT/solution saline) et à l'aide d'une ANOVA à un facteur que l'on fait suivre par un test *post-hoc* de Tukey (# : p<0,05, ## : p<0,01 et ### : p<0,001 par rapport au groupe CMT1A/solution saline ; $ : p<0,05, $$ : p<0,01 et $$$ : p<0,001 par rapport au groupe CMT1A/Nano). | | | | | | |

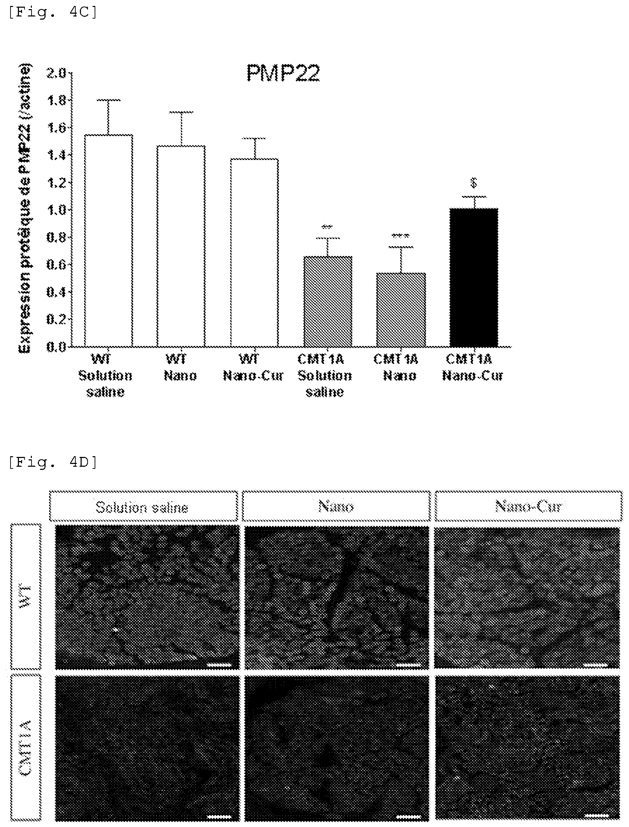

L'expression des protéines Myelin Protein Zero (MPZ), Myelin Basic Protein (MBP) et Peripheral Myelin Protein 22 (PMP22) a été évaluée dans des homogénats de nerf sciatique à W8 (Figures 4A-C). Aucune différence significative n'a été observée dans l'expression de ces protéines entre tous les groupes WT. Par comparaison aux rats WT/solution saline, une diminution significative de l'expression de MPZ a été observée dans tous les groupes CMT1A (p < 0,001) (Figure 4A). Aussi, une expression accrue de MPZ a été observée chez les rats CMT1A/Nano-Cur (p-<-0,05) par rapport à des rats CMT1A/solution saline (Figure 4A). De manière similaire, une diminution de l'expression de MBP a également été observée dans tous les groupes CMT1A (p < 0,001) par comparaison aux rats WT/solution saline (Figure 4B). De plus, une diminution de l'expression de PMP22 a été observée chez les rats CMT1A/solution saline (p < 0,001) et les rats CMT1A/Nano (p < 0,001) par comparaison aux rats WT. Cependant, aucune différence dans les taux d'expression de PMP22 n'a été observée entre les animaux WT/solution saline et les animaux CMT1A/Nano-Cur (Figure 4C). Pour finir, une expression accrue de PMP22 a été également observée chez les rats CMT1A/Nano-Cur (p-<-0,05) par rapport à des rats CMT1A/Nano. Ces résultats ont été confirmés par marquage immunofluorescent de PMP22 sur des coupes de nerf sciatique à W8 (Figure 4D).

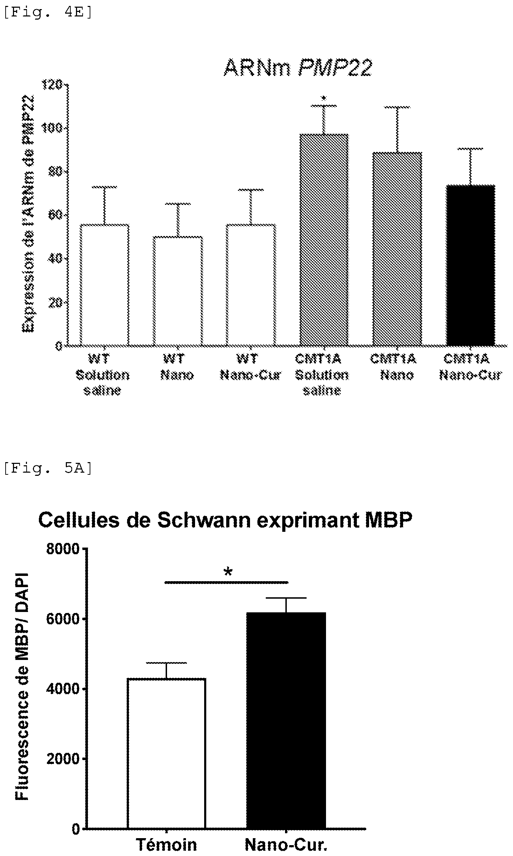

Ainsi, l'expression de l'ARNm de PMP22 a été évaluée dans des homogénats de nerf sciatique à W8 (Figure 4E). Aucune différence significative n'a été observée dans l'expression de l'ARNm de PMP22 entre tous les groupes WT. Cependant, une augmentation significative de l'expression de l'ARNm de PMP22 a été identifiée chez les rats CMT1A/solution saline (p<0,01) par comparaison aux animaux WT/solution saline. De manière similaire, une augmentation de l'expression de l'ARNm de PMP22 a été observée chez les rats CMT1A/Nano par comparaison aux rats WT/Nano. En revanche, aucune différence significative n'a été observée entre les groupes WT/solution saline et CMT1A/Nano-Cur. Ces résultats suggèrent que le traitement par Nano-Cur favorise la myélinisation chez les rats CMT1A.

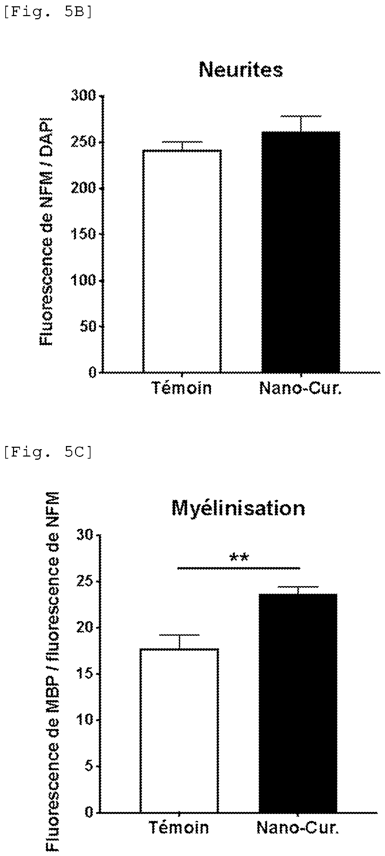

La myélinisation a été évaluée *in vitro* par un marquage immunofluorescent de la MBP (marquage de la myéline) et NFM (marquage des neurites) à partir de co-cultures de cellules de Schwann et de neurones provenant de rats de type WT (données non montrées). Ces résultats indiquent que le traitement par Nano-Cur induit une augmentation significative de l'expression de MBP *in vitro* par comparaison à la condition contrôle sans traitement (p < 0,05) (Figure 5A). Cependant, il n'a pas été observé d'effet significatif du traitement par Nano-Cur sur l'expression de NFM par comparaison à la condition contrôle (Figure 5B). De plus, l'analyse des données du ratio MBP/NFM indique une augmentation significative de la myélinisation avec le traitement par Nano-Cur (p<0,01) (Figure 5C). Ces résultats suggèrent que le traitement par Nano-Cur améliore la myélinisation *in vitro,* à partir de co-cultures de cellules de Schwann et de neurones.

### Exemple 5 : Le traitement par Nano-Cur diminue le stress oxydant et induit l'expression d'enzymes antioxydantes chez les rats CMT1A

Le stress oxydant a été évalué par quantification des espèces réactives de l'oxygène (ROS) à partir de coupes de nerf sciatique à W8 (Figure 6A). Si on se réfère à la Figure 6A, une augmentation significative de la teneur en ROS totales a été observée chez les rats CMT1A/solution saline (p<0,05) et les rats CMT1A/Nano (p<0,01) par rapport aux rats WT/solution saline. De façon intéressante, dans les nerfs sciatiques de rats CMT1A traités avec Nano-Cur, aucune différence significative n'a été observée par comparaison au groupe WT/solution saline. De plus, une diminution significative de la teneur en ROS a été observée chez les CMT1A/Nano-Cur par rapport aux rats CMT1A/solution saline (p<0,05) et aux rats CMT1A/Nano (p<0,01). Ces résultats indiquent un effet bénéfique du traitement par Nano-Cur sur le stress oxydant dans les nerfs sciatiques de rats CMT1A.

L'expression de la protéine Nrf2, un facteur de transcription impliqué dans la production d'enzymes antioxydantes, a aussi été évaluée à partir de nerfs sciatiques de rats (Figure 6B). Si on se réfère à la Figure 6B, aucune différence significative de l'expression de Nrf2 n'a été observée entre les groupes CMT1A/solution saline et CMT1A/Nano par comparaison avec le groupe WT/solution saline. Les résultats ont montré une expression accrue de Nrf2 chez les rats CMT1A/Nano-Cur par comparaison aux rats WT/solution saline (p<0,01), CMT1A/solution saline (p<0,05) et CMT1A/Nano (p<0,05), suggérant un effet antioxydant de Nano-Cur.

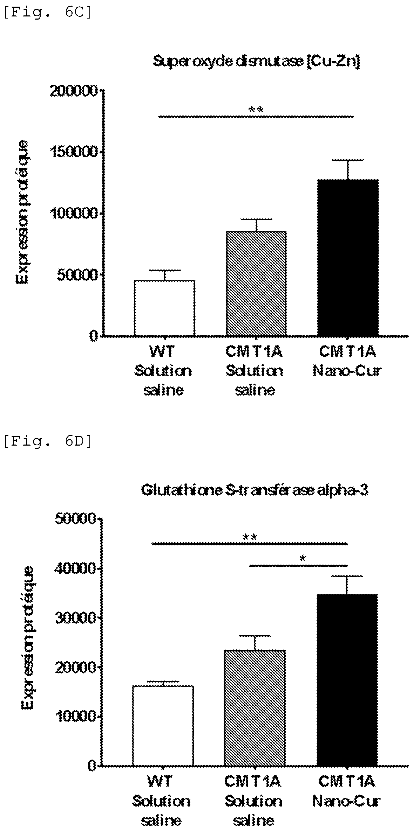

L'expression de certaines enzymes antioxydantes (superoxyde dismutase [Cu-Zn] et glutathion S-transférase alpha-3) a également été évaluée par analyse protéomique (spectrométrie de masse) des homogénats de nerf sciatique à W8 (Figures 6C-6D). Les analyses ont indiqué une augmentation significative de l'expression des principales enzymes antioxydantes par rapport aux rats WT/Saline (superoxyde dismutase[Cu-Zn] : p<0,01 ; glutathion S-transférase). De plus, une augmentation significative de l'expression de l'enzyme antioxydante glutathion S-transférase alpha-3 a été observée chez les animaux CMT1A/Nano-Cur comparativement aux rats CMT1A/Saline (p<0,05)(Figure 6D). Ces résultats suggèrent que le traitement Nano-Cur augmente l'expression des enzymes antioxydantes dans les nerfs sciatiques des rats CMT1A.

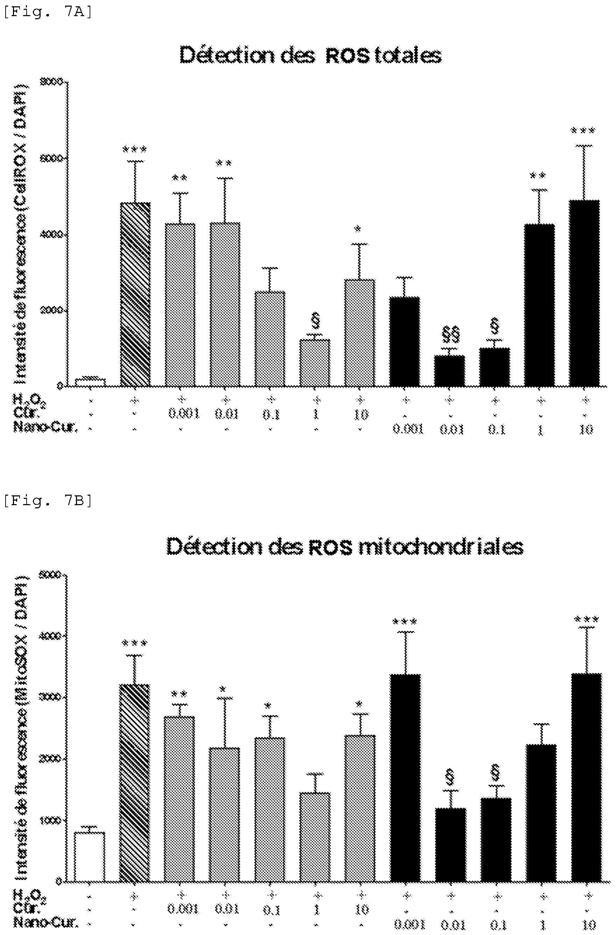

Ensuite, les effets de la curcumine seule et de Nano-Cur sur le stress oxydant induit par H₂O₂ ont été étudiés *in vitro* à partir de cultures primaires de cellules de Schwann de rats WT. Pour étudier et identifier la dose la plus efficace, des doses de 0,001, 0,01, 0,1, 1 et 10 µM de curcumine seule ou de Nano-Cur ont été testées. Ces résultats indiquent que le traitement par H₂O₂ (0,1 mM) a induit une augmentation significative des teneurs en ROS totales et mitochondriales et une diminution du Δψm (indice de l'intégrité de la membrane mitochondriale) par comparaison aux groupes témoins (non soumis au traitement par H₂O₂₎ (p < 0,001) (Figures 7A-C). Si l'on se réfère à la Figure 7A, le traitement par la curcumine seule à 1 µM a réduit la teneur en ROS totales induite par H₂O₂ (p<0,05), alors que des doses de 0,001, 0,01 et 10 µM n'ont pas eu d'effet antioxydant. Pour le traitement par Nano-Cur, des doses de 0,01 µM (p<0,01) et 0,1 µM (p<0,05) ont montré le plus grand effet antioxydant.

Concernant la production de ROS mitochondriales, la dose de 1 µM de curcumine seule a permis de restaurer des valeurs proches de celles du groupe témoin (non soumis au traitement par H₂O₂) (Figure 7B). Cependant, le traitement Nano-Cur a permis de réduire significativement les ROS mitochondriales induites par H₂O₂ aux concentrations beaucoup plus faibles de 0,01 µM (p<0,05) et de 0,1 µM (p<0,05).

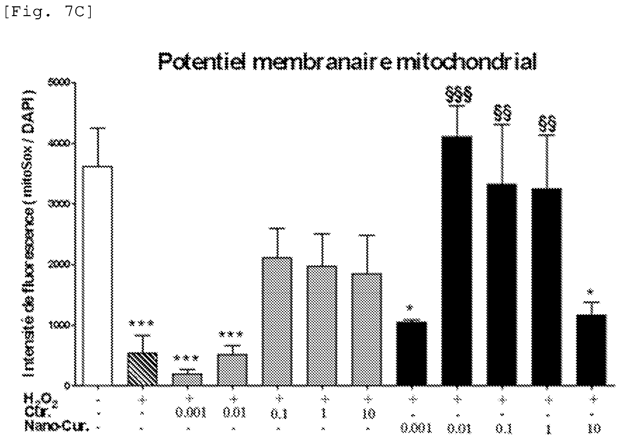

D'une manière similaire, si l'on se réfère à la Figure 7C, aux concentrations de 0,001 µM et 0,01 µM la curcumine seule n'a pas permis de réduire la baisse du Δψm induite par H₂O₂, En revanche, des doses de Nano-Cur de 0,01 µM (p<0,001), 0,1 µM (p<0,01) et 1 µM (p<0,01) ont augmenté significativement le Δψm par rapport au groupe H₂O₂. Ces résultats suggèrent que de faibles doses de Nano-Cur (0,01 et 0,1 µM) ont l'effet antioxydant le plus puissant.

Pris conjointement, tous ces résultats contribuent à montrer que le traitement par Nano-Cur conduit à une amélioration de la lésion qui se développe chez les rats transgéniques modèles de CMT1A. En effet, le traitement par Nano-Cur améliore l'équilibre et la force d'agrippement et limite la perte de la sensibilité tactile et thermique des rats CMT1A. Le traitement par Nano-Cur utilisé dans l'étude a augmenté la MNCV mais pas l'amplitude des potentiels d'action musculaires. De plus, le traitement par Nano-Cur a permis de détecter les réflexes H et ainsi le calcul de la SNCV chez les rats CMT1A, bien que ce signal reste faible. Il est communément accepté que la MNCV est liée à l'intégrité et à l'épaisseur de la gaine de myéline. Le traitement par Nano-Cur a permis de limiter la démyélinisation/dysmyélinisation des nerfs chez les animaux CMT1A, ces résultats étant supportés par une expression plus élevée des protéines de la myéline compacte chez les animaux traités.

Une augmentation du stress oxydant a été décrit chez les patients CMT1A (Chahbouni et al., 2017). Cependant, jusqu'à ce jour, le stress oxydant n'avait pas été étudié dans le modèle du rat CMT1A. Il est reconnu qu'une augmentation excessive des ROS est délétère pour les cellules, en particulier via les dommages à l'ADN, aux protéines et aux lipides. De plus, en raison de la structure multilamellaire de la myéline, les cellules de Schwann sont particulièrement sensibles à la lipopéroxydation. Ainsi, il semble approprié de chercher à réduire le stress oxydant dans la maladie de Charcot-Marie-Tooth de type 1A. Les résultats présentés contribuent à démontrer qu'une dose faible et continue de curcumine, délivrée par des nanocristaux de cellulose, représente une thérapie prometteuse pour les neuropathies périphériques, notamment la maladie de Charcot-Marie-Tooth de type 1A, sous la forme non limitative d'hydrogels, d'implants sous-cutanés, de pompe implantable ou de conduit nerveux biofonctionnalisé implanté.

### Références bibliographiques

- Agthong, S., Kaewsema, A., Charoensub, T., 2015. Curcumin Ameliorates Functional and Structural Abnormalities in Cisplatin-induced Neuropathy. Exp. Neurobiol. 24, 139-145.
- Al Moundhri, M.S., Al-Salam, S., Al Mahrouqee, A., Beegam, S., Ali, B.H., 2013. The effect of curcumin on oxaliplatin and cisplatin neurotoxicity in rats: some behavioral,biochemical, and histopathological studies. J. Med. Toxicol. Off. J. Am. Coll. Med. Toxicol. 9, 25-33.
- Caillaud M, Chantemargue B, Richard L, Vignaud L, Favreau F, Faye P-A, et al. Local low dose curcumin treatment improves functional recovery and remyelination in a rat model of sciatic nerve crush through inhibition of oxidative stress. Neuropharmacology 2018; 139: 98-116.
- Chahbouni M, Lôpez MDS, Molina-Carballo A, de Haro T, Muñoz-Hoyos A, Fernândez-Ortiz M, et al. Melatonin Treatment Reduces Oxidative Damage and Normalizes Plasma Pro-Inflammatory Cytokines in Patients Suffering from Charcot-Marie-Tooth Neuropathy: A Pilot Study in Three Children. Mol. Basel Switz. 2017; 22(10), 1728.
- Daugherty, D.J., Marquez, A., Calcutt, N.A., Schubert, D., 2018. A novel curcumin derivative for the treatment of diabetic neuropathy. Neuropharmacology 129, 26-35.
- Han, F., Luo, B., Shi, R., Han, C., Zhang, Z., Xiong, J., Jiang, M., Zhang, Z., 2014. Curcumin ameliorates rat experimental autoimmune neuritis. J. Neurosci. Res. 92, 743-750.
- Kandhare, A.D., Raygude, K.S., Ghosh, P., Ghule, A.E., Bodhankar, S.L., 2012. Therapeutic role of curcumin in prevention of biochemical and behavioral aberration induced by alcoholic neuropathy in laboratory animals. Neurosci. Lett. 511, 18-22.
- Kaur, M., Singh, A., Kumar, B., Singh, S.K., Bhatia, A., Gulati, M., Prakash, T., Bawa, P., Malik, A.H., 2017. Protective effect of co-administration of curcumin and sildenafil in alcohol induced neuropathy in rats. Eur. J. Pharmacol. 805, 58-66.
- Khajavi M, Inoue K, Wiszniewski W, Ohyama T, Snipes GJ, Lupski JR. Curcumin treatment abrogates endoplasmic reticulum retention and aggregation-induced apoptosis associated with neuropathy-causing myelin protein zero-truncating mutants. Am. J. Hum. Genet. 2005; 77: 841-850.
- Khajavi M, Shiga K, Wiszniewski W, He F, Shaw CA, Yan J, et al. Oral curcumin mitigates the clinical and neuropathologic phenotype of the Trembler-J mouse: a potential therapy for inherited neuropathy. Am. J. Hum. Genet. 2007; 81: 438-453.
- Liu, G.-M., Xu, K., Li, J., Luo, Y.-G., 2016. Curcumin upregulates S100 expression and improves regeneration of the sciatic nerve following its complete amputation in mice. Neural Regen. Res. 11, 1304-1311.
- Lv, J., Cao, L., Zhang, R., Bai, F., Wei, P., 2018. A curcumin derivative J147 ameliorates diabetic peripheral neuropathy in streptozotocin (STZ)-induced DPN rat models through negative regulation AMPK on TRPA1. Acta Cir. Bras. 33, 533-541.
- Mohammadi, R., Mahmoodi, H., 2013. Improvement of peripheral nerve regeneration following nerve repair by silicone tube filled with curcumin: a preliminary study in the rat model. Int. J. Surg. Lond. Engl. 11, 819-825.
- Ndong Ntoutoume GMA, Granet R, Mbakidi JP, Brégier F, Léger DY, Fidanzi-Dugas C, et al. Development of curcumin-cyclodextrin/cellulose nanocrystals complexes: New anticancer drug delivery systems. Bioorg. Med. Chem. Lett. 2016; 26: 941-945.
- Ndong Ntoutoume GMA, Grassot V, Brégier F, Chabanais J, Petit J-M, Granet R, et al. PEI-cellulose nanocrystal hybrids as efficient siRNA delivery agents-Synthesis, physicochemical characterization and in vitro evaluation. Carbohydr. Polym. 2017; 164: 258-267.
- Nobbio L, Vigo T, Abbruzzese M, Levi G, Brancolini C, Mantero S, et al. Impairment of PMP22 transgenic Schwann cells differentiation in culture: implications for Charcot-Marie-Tooth type 1A disease. Neurobiol. Dis. 2004; 16: 263-273.
- Okamoto Y, Pehlivan D, Wiszniewski W, Beck CR, Snipes GJ, Lupski JR, et al. Curcumin facilitates a transitory cellular stress response in Trembler-J mice. Hum. Mol. Genet. 2013; 22: 4698-4705.
- Prasad, S., Tyagi, A.K., Aggarwal, B.B., 2014. Recent developments in delivery, bioavailability, absorption and metabolism of curcumin: the golden pigment from golden spice. Cancer Res. Treat. Off. J. Korean Cancer Assoc. 46, 2-18.
- Sereda M, Griffiths I, Pühlhofer A, Stewart H, Rossner MJ, Zimmerman F, et al. A transgenic rat model of Charcot-Marie-Tooth disease. Neuron 1996; 16: 1049-1060.
- Sharma, R.A., Euden, S.A., Platton, S.L., Cooke, D.N., Shafayat, A., Hewitt, H.R., Marczylo, T.H., Morgan, B., Hemingway, D., Plummer, S.M., Pirmohamed, M., Gescher, A.J., Steward, W.P., 2004. Phase I clinical trial of oral curcumin: biomarkers of systemic activity and compliance. Clin. Cancer Res. Off. J. Am. Assoc. Cancer Res. 10, 6847-6854.
- Shoba, G., Joy, D., Joseph, T., Majeed, M., Rajendran, R., Srinivas, P.S., 1998. Influence of piperine on the pharmacokinetics of curcumin in animals and human volunteers. Planta Med. 64, 353-356.
- Wahlström, B. and Blennow, G., 1978. A study on the fate of Curcumin in the rat. Acta pharmacologica et toxicologica, Vol. 43, Issue 2, 86-92.
- Yang, K.-Y., Lin, L.-C., Tseng, T.-Y., Wang, S.-C., Tsai, T.-H., 2007. Oral bioavailability of curcumin in rat and the herbal analysis from Curcuma longa by LC-MS/MS. J. Chromatogr. B Analyt. Technol. Biomed. Life. Sci. 853, 183-189.

## Revendications

1. - Complexe comprenant :
- des nanocristaux de cellulose ;
- au moins une molécule de β-cyclodextrine ;
- au moins une molécule de curcumine,
pour une utilisation dans le traitement de tout type de neuropathies périphériques.

2. - Complexe pour son utilisation selon la revendication 1, **caractérisé par le fait que** la neuropathie périphérique est une maladie de Charcot-Marie-Tooth.

3. - Complexe pour son utilisation selon l'une des revendications 1 ou 2, **caractérisé par le fait que** la neuropathie périphérique est la maladie de Charcot-Marie-Tooth de type 1A.

4. - Complexe pour son utilisation selon la revendication 1, **caractérisé par le fait que** la neuropathie périphérique est liée à une lésion traumatique.

5. - Complexe pour son utilisation selon l'une quelconque des revendications précédentes, **caractérisé par le fait que** le complexe est destiné à être administré à un sujet selon une quantité thérapeutiquement efficace.

6. - Complexe pour son utilisation selon l'une quelconque des revendications précédentes, **caractérisé par le fait que** la quantité thérapeutiquement efficace est comprise entre 0,02 et 200 mg/kg/jour de curcumine, en particulier entre 0,1 et 1 mg/kg/jour, plus particulièrement entre 0,1 et 0,3 mg/kg/jour.

7. - Complexe pour son utilisation selon l'une quelconque des revendications précédentes, **caractérisé par le fait que** le complexe est destiné à être administré à un sujet par voie injectable, de préférence sous-cutanée, intramusculaire, intraveineuse ou périneurale, par voie orale, par voie transdermique ou dans un conduit nerveux biofonctionnalisé implanté.

8. - Composition pharmaceutique comprenant :
- au moins le complexe selon l'une quelconque des revendications 1 à 7 ;
- au moins un excipient pharmaceutiquement acceptable
pour une utilisation dans le traitement de tout type de neuropathies périphériques.

9. - Composition pharmaceutique pour son utilisation selon la revendication 8, **caractérisée en ce qu'**elle est sous la forme d'un hydrogel.

10. - Complexe pour son utilisation selon l'une quelconque des revendications 1 à 7, ou composition pharmaceutique pour son utilisation selon la revendication 8, **caractérisée en ce que** le complexe ou la composition est administré(e) via un implant sous-cutané, une pompe implantable, ou un conduit nerveux biofonctionnalisé implanté.

## Patentansprüche

1. Komplex, umfassend:
- Cellulose-Nanokristalle,
- wenigstens ein Molekül β-Cyclodextrin,
- wenigstens ein Molekül Curcumin,
zur Verwendung bei der Behandlung aller Arten von peripheren Neuropathien.

2. Komplex zur Verwendung nach Anspruch 1, **dadurch gekennzeichnet, dass** die periphere Neuropathie eine Charcot-Marie-Tooth-Krankheit ist.

3. Komplex zur Verwendung nach einem der Ansprüche 1 oder 2, **dadurch gekennzeichnet, dass** die periphere Neuropathie die Charcot-Marie-Tooth-Krankheit vom Typ 1A ist.

4. Komplex zur Verwendung nach Anspruch 1, **dadurch gekennzeichnet, dass** die periphere Neuropathie mit einer traumatischen Verletzung in Verbindung steht.

5. Komplex zur Verwendung nach einem der vorhergehenden Ansprüche, **dadurch gekennzeichnet, dass** der Komplex dazu bestimmt ist, einem Subjekt in einer therapeutisch wirksamen Menge verabreicht zu werden.

6. Komplex zur Verwendung nach einem der vorhergehenden Ansprüche, **dadurch gekennzeichnet, dass** die therapeutisch wirksame Menge zwischen 0,02 und 200 mg/kg/Tag Curcumin liegt, insbesondere zwischen 0,1 und 1 mg/kg/Tag, ganz besonders zwischen 0,1 und 0,3 mg/kg/Tag.

7. Komplex zur Verwendung nach einem der vorhergehenden Ansprüche, **dadurch gekennzeichnet, dass** der Komplex dazu bestimmt ist, einem Subjekt durch Injektion, vorzugsweise subkutan, intramuskulär, intravenös oder perineural, oral, transdermal oder in einen implantierten biofunktionalisierten Nervenkanal verabreicht zu werden.

8. Pharmazeutische Zusammensetzung, umfassend:
- wenigstens den Komplex nach einem der Ansprüche 1 bis 7,
- wenigstens einen pharmazeutisch akzeptablen Hilfsstoff,
zur Verwendung bei der Behandlung aller Arten von peripheren Neuropathien.

9. Pharmazeutische Zusammensetzung zur Verwendung nach Anspruch 8, **dadurch gekennzeichnet, dass** sie in Form eines Hydrogels vorliegt.

10. Komplex zur Verwendung nach einem der Ansprüche 1 bis 7 oder pharmazeutische Zusammensetzung zur Verwendung nach Anspruch 8, **dadurch gekennzeichnet, dass** der Komplex oder die Zusammensetzung über ein subkutanes Implantat, eine implantierbare Pumpe oder einen implantierten biofunktionalisierten Nervenkanal verabreicht wird.

## Claims

1. - A complex comprising:
- cellulose nano crystals;
- at least one β-cyclodextrin molecule;
- at least one curcumin molecule,
for use in the treatment of any type of peripheral neuropathies.

2. - The complex for use according to claim 1, **characterised in that** the peripheral neuropathy is Charcot-Marie-Tooth disease.

3. - The complex for use according to one of claims 1 or 2, **characterised in that** the peripheral neuropathy is type 1A Charcot-Marie-Tooth disease.

4. - The complex for use according to claim 1, **characterised in that** the peripheral neuropathy is related to a traumatic injury.

5. - The complex for use according to any of the preceding claims, **characterised in that** the complex is to be administered to a subject in a therapeutically effective amount.

6. - The complex for use according to any of the preceding claims, **characterised in that** the therapeutically effective amount is between 0.02 and 200 mg/kg/day of curcumin, in particular between 0.1 and 1 mg/kg/day, more particularly between 0.1 and 0.3 mg/kg/day.

7. - The complex for use according to any of the preceding claims, **characterised in that** the complex is to be administered to a subject by injectable route, preferably subcutaneous, intramuscular, intravenous or perineural, by oral route, by transdermal route or in an implanted biofunctionalised nerve duct.

8. - A pharmaceutical composition comprising:
- at least the complex according to any of claims 1 to 7;
- at least one pharmaceutically acceptable excipient
for use in the treatment of any type of peripheral neuropathies.

9. - The pharmaceutical composition for use according to claim 8, **characterised in that** it is in the form of a hydrogel.

10. - The complex for use according to any of claims 1 to 7, or the pharmaceutical composition for use according to claim 8, **characterised in that** the complex or composition is administered via a subcutaneous implant, an implantable pump, or an implanted biofunctionalised nerve duct.
